# EUROPEAN PATENT SPECIFICATION

(11) **EP 0 292 621 B1**
(45) Date of publication and mention of the grant of the patent: **14.12.1994**
(21) Application number: 87304619.7
(22) Date of filing: 26.05.1987
(51) Int. Cl.: A61N 5/06, A61B 17/36

(54) **Contact or insertion laser probe having wide angle radiation**
Mit einem breiten Strahlenwinkel versehene Lasersonde für Kontaktierung oder Einfügung
Embout laser de contact ou d'insertion à angle large de radiation

(43) Date of publication of application: 30.11.1988
(73) Proprietor: Surgical Laser Technologies, Inc., Malvern Pennsylvania 19355 (US)
(72) Inventor: Daikuzono, Norio, Ichikawa-shi Chiba-ken Tokyo (JP)
(74) Representative: Lyons, Andrew John

(56) References cited:
- EP-A- 0 161 606
- WO-A-85/05262
- GB-A- 2 154 761
- OPTICS AND LASER TECHNOLOGY, vol. 16, no. 6, February 1984, pages 40-44, Butterworth & Co. Ltd, Exeter, GB; H. FUJII et al.: "Light scattering properties of a rough-ended optical fibre"

## Description

This invention relates to a medical probe used for effecting hyperthermia, coagulation or hemostasis with respect to tissues of human or animal organisms by laser irradiation thereof.

There have been recent advances in the field of laser surgery particularly in connection with YAG lasers and the use of contact laser probes wherein, contrary to established convention, the probe may be brought into direct contact with the tissue.

Whether of a contact or non-contact variety, it is known that penetration of laser energy into tissue is limited and, therefore, laser treatment of sub-surface tissue requires removal or cutting of the overlying surface tissue. This characteristic of laser surgery does not represent a problem during many procedures, for example incision, where it is the very purpose of the procedure to cut through the surface layers of tissue.

On the other hand, there are other procedures in which it is desirable to laser irradiate tissue lying below the surface without irreparably damaging the tissue thereabove. One such procedure, known as PRT (photoradiation therapy), is reported to selectively break cancerous tissue when the tissue has been pretreated with a photosensitizer. (See Doughterty, T.J. et al.: Photoradiation Therapy for the Treatment of Malignant Tumors. Cancer Res., 38: 2628 - 2635, 1978). PRT involves the intravenous injection of a photosensitizer, generally hematoporphyrin derivatives (HpD), approximately 48 to 72 hours before tissue irradiation by an argon dye laser. It has been observed that cancerous tissues are broken while the normal tissues remain virtually unaffected.

It will be appreciated that PRT treatment requires the irradiation of the entire cancerous area, such area generally lying well below the tissue surface. It is therefore preferable, if not necessary, that the laser probe be inserted into the tissue so that the laser beam may sufficiently react with the photosensitizer. Further, it is necessary that the tip exhibit a broad radiation pattern to insure the widest possible illumination of presensitized cancerous tissue.

Known laser probes, however, emit laser energy only from the tip region of the probe and, then, only in a relatively narrow beam directed substantially outwardly and downwardly from the probe tip. When such a tip is inserted into the cancerous tissue, two problems are seen to occur. First, the relatively narrow illumination pattern, for example 8 to 14 degrees, results in a correspondingly limited tissue treatment region. As it is necessary to expose tissue for about 15 to 35 minutes, it takes considerable time to irradiate any but the smallest areas requiring treatment. The second problem encountered with conventional probes is the charring of tissue immediately adjacent the probe tip due to the high power density of the concentrated laser beam in that region. Charring prevents the laser beam from penetrating the charred portion of the tissue, in turn, remarkably lowering the overall penetration of the laser beam.

In addition to PRT, a probe which is capable of widely diffusing the laser beam may advantageously be utilized for the treatment of gastric cancer, ulcer, gastric or intestinal haemorrhage, or local hyperthermia. For the local hyperthermia, Nd-YAG laser may also be employed.

Optics and Laser Technology, Vol 16, no. 6, Feb 1984, pages 40-44, discloses a medical laser probe comprising an optic fiber guide having a roughened tip in order to achieve wide-angle irradiation of a laser beam.

It is therefore an object of the present invention to provide a probe which is capable of irradiating a wide surface area in a diffused irradiation mode.

It is another object of the present invention to provide a probe which is capable of emitting laser beam from all over the irradiating surface area with substantially uniform power density.

The present invention is defined with claim 1.

The preferred arrangement of the present invention is a conically shaped probe of quartz, sapphire, or diamond. While the design of such a probe can be varied to either facilitate or inhibit radiation from the sides of the conical probe tip; the resulting radiation pattern will, absent the teachings of the present invention, remain substantially outward along the axis of the probe. The area of illumination remains limited.

The present invention, therefore, relates to a laser probe having a substantially wider radiation pattern, in fact, a pattern in which both radial sideways as well as rearward backscattering may be achieved in addition to nominal forward radiation. The present probe tip achieves not only broader tissue illumination, but importantly, it does so at substantially more uniform laser energy densities thereby greatly minimizing the likelihood of tissue charring. In particular, the present probe tip is characterized by an uneven or roughened surface having recesses therein ranging in depth from about 10 to 60 µm. As a result, some of the laser energy which reaches this surface is reflected while the remainder is radiated generally to the tissue adjacent thereto.

Thus, the laser beam impinging on this surface from within the tip is, in part, refracted directly to the tissue and, in part, further reflected irregularly within the concaved portions of the uneven roughened surface. This scattering of the beam occurs over the entire roughened surface of the probe so that the emitted laser energy impinges the tissue substantially from all directions thereby broadening the radiation area and increasing penetration into the tissue by lowering local charring of the tissue.

In accordance with the present invention, there is further provided a medical laser probe adapted to transmit laser energy propagated through a waveguide to provide the same to the tissue, which probe comprises a laser diffusing member provided so as to surround a tip end portion of the waveguide for covering the laser beam radiated from the tip end of the waveguide, said laser diffusing member having, on the inner surface thereof, a frosted or roughened face, over the area of the inner surface which receives the laser beam.

This form of the probe is characterized in that the frosted or roughened surface is formed on the inner face of the laser diffusing member. The laser beam is partially transmitted through the inner frosted or roughened surface and partially reflected irregularly to reach another portion of the probe where the laser beam is transmitted or again reflected irregularly. As a result, laser energy is radiated from a wide outer surface of the probe at substantially uniform power density. In this case, the laser radiating area may be wide, for example, on a round surface of large diameter so that it may be used in a mode contacting with the surface of the tissue for effecting the coagulation of the tissue.

The invention will now be further described, by way of example only, with reference to the accompanying drawings, in which:
Figure 1 is an elevation view, part in section, of the probe of the present invention and a holding member therefor;
Figure 2 is an elevation view showing the probe of Figure 1 inserted into the tissue and irradiating the tissue adjacent thereto;
Figure 3 is an enlarged sectional view of the tip end portion of the probe;
Figure 4 is a further enlarged sectional view of the surface portion of the probe showing the diffusion modes of the laser beam;
Figure 5 is an elevation view of a non-diffusing probe inserted into the tissue and irradiating the tissue immediately below the probe tip;
Figure 6 is a distribution diagram comparing the radiated power densities of the present probe against non-diffusing probes;
Figure 7 is an elevation view of one example of the probe of the present invention showing the dimensions thereof; and
Figure 8 is an elevation view, shown in section, of another embodiment of the probe according to the present invention.

### DESCRIPTION OF THE PREFERRED EMBODIMENT

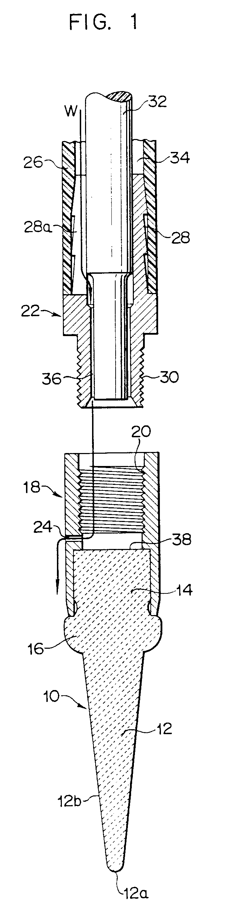

Figure 1 is a longitudinal sectional view of a probe 10 according to the present invention which is mounted at the terminal or output end portion of a laser optical waveguide fiber 32.

Probe 10 is made of a laser transmissive material such as a natural or artificial ceramic material, for example, sapphire, quartz or diamond. Polymeric materials may also be employed. In the embodiment as illustrated, the probe 10 comprises a conically tapered laser diffusing portion 12 having, at the tip end thereof, a semispherical portion 12a and a main body portion 14. The laser diffusing portion 12 and the main body portion 14 are formed integrally with each other and a flange 16 is formed between the laser diffusing portion 12 and the main body portion 14. The probe 10 is fitted in a cylindrical female connector 18 and fixed integrally thereto by caulking the mating surfaces thereof or using a ceramic type adhesive between the mating surfaces. The female connector 18 has, on the internal surface thereof, a thread 20 which is adapted to mate with complementary threads 30 of a male connector 22. The female connector 18 has two holes 24 through the cylindrical connector wall which facilitate the passage of cooling water W or other fluids therethrough. The two holes are circumferentially disposed at angular spaces of 180^{o} although only one of them is shown in Figure 1.

On the other hand, the male connector 22 is pressedly fitted into a flexible tube of jacket 26 fabricated of, for example, Teflon (trademark). For this press fitting, the male connector 22 has stepped portions 28 at the base portion of the male connector 22 by which the male connector 22 is firmly held within jacket 26 so as to prevent the former from being disengaged from the latter. As noted, male connector 22 is externally threaded at 30 to mate with the internal thread 20 of the female connector 18.

An optical fiber 32 for transmitting the laser beam is inserted in the male connector 22. The optical fiber 32 is disposed concentrically within the jacket 26, leaving a gap 34 therebetween for passage of cooling water or other fluids. Although the fiber 32 is closely fitted in the male connector 22 adjacent the stepped portion of the male connector, the stepped portion 28 has for example two slits 28a formed circumferentially at angular spaces of 180^{o} for letting the cooling water W pass therethrough. A passage 36 for the cooling water W is further provided between the inner face of the tip end portion of the male connector 22 and the optical fiber 32. The assembled tip may then be inserted into an endoscope or otherwise positioned as required for the laser surgical procedure to be performed.

The fiber 32 is optically connected to a laser generating unit (not shown). According to necessity, cooling water W is fed through the gap 34; slit 28a; passage 36 then, in turn, discharged through the opening 24 to cool the tissue to be treated.

The laser beam from the laser generating unit is guided through the optical fiber 32 and coupled from the end thereof into the base 38 of probe 10. The laser beam is thereafter emitted, as discussed in more detail below, from the diffusing portion 12 of the tip to the tissue M to be treated.

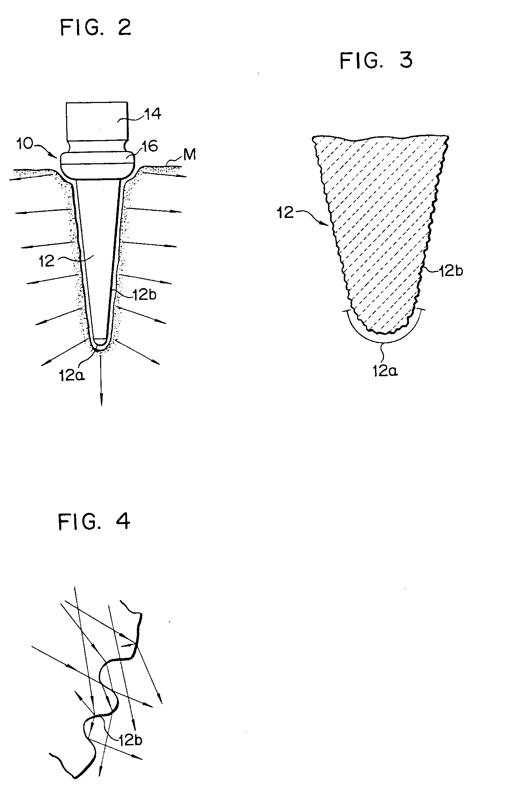

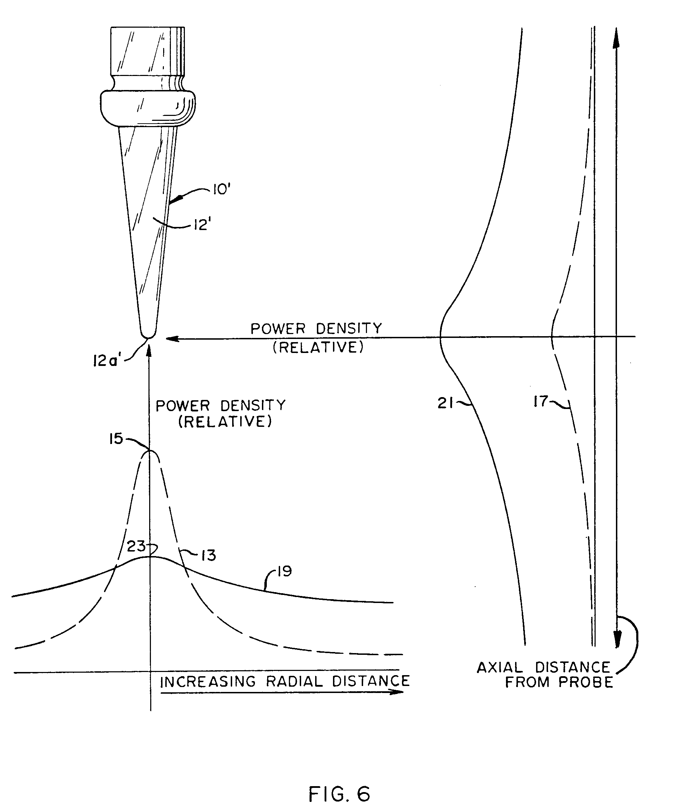

Figure 2 illustrates the dispersion and diffusion of laser beam when the probe of the present invention is employed. As will be described later, the outer surface of the laser diffusing portion 12 of the probe 10 is frosted or roughened, thereby widely dispersing the laser energy as illustrated in Figure 2.

Figure 6 best illustrates the comparative radiation energy densities between a prior art probe tip and the probe tip of the present invention. Probe tip 11' may be of either type. Curve 13 represents the laser energy or power density distribution in a plane spaced below the probe tip and perpendicular to the longitudinal axis thereof. More specifically, the power density is shown to be greatest at 15, a point along the extended longitudinal axis of the probe tip, and to diminish rapidly as the distance from this axis is increased. Curve 17 very roughly approximates the laser power density distribution in a cylindrical surface surrounding the probe and having a common axis thereto. This curve essentially depicts the radial component of laser radiation which, as can be seen, is extremely low in a conventional tip due to the fact that substantially all of the laser energy is radiated longitudinally downwardly from tip 12a'. Curve 17, therefore, is merely intended to depict this low level of radial radiation rather than to accurately illustrate the actual power distribution.

Curves 19 and 21 represent the laser power density distributions for the probe of the present invention corresponding, respectively, to prior art probe curves 13 and 17. As can be seen from curve 19, the peak power density 23 along tee probe center axis is substantially lower than that of the prior art tip and, importantly, the power density drops less rapidly as a function of distance from this axis. This produces a broad area of relatively uniform laser illumination while simultaneously avoiding the excessive on-axis power densities which result in tissue charring. Curve 21 reveals that substantial laser energy is emitted radially from the probe of the present invention and, further, that such radiation is not limited to the tip region 12a', but occurs along the entire length of the conical diffusing portion 12' of the probe. However, as discussed in more detail below, the laser power distribution depicted by curve 21 is, in reality, achieved by the random refraction and scattering of laser energy within the probe tip and, therefore, this radiation, while illuminating tissue along the side of the tip, is not necessarily emitted radially therefrom.

As best shown in Figures 3 and 4, the outer surface of the laser diffusing portion 12 is frosted or roughened to form an uneven, pseudo-random contour defined by generally convex protuberances and concave recesses. The surface irregularity or recess depth is between 10 to 60 um. The frosting or roughening process is carried out by use of a computer controlled grinding wheel. More specifically, the probe undergoing surface treatment is rotated and then brought into contact with a diamond grindstone. The grindstone traces the unroughened contour of the probe, commencing from the tip 12a of the probe, as far rearward along the conical surface as desired to define the diffusing portion 12 thereof. The computer controls, in a conventional manner, the position and speed of travel of the grindstone. In one preferred arrangement, a grindstone having particles of between 10 to 20 um is utilized while the grindstone is moved along the probe between 3 and 6 mm/second. A roughened surface condition defined by approximately 10um recesses results.

It will be appreciated that the depth of the recesses may be varied by utilizing grindstones of appropriate diamond particle size. However, as the recess depth is lowered, particularly below the preferred range set forth above, an increasing percentage of the laser energy will be internally reflected within the probe tip thereby resulting in a correspondingly lesser amount laser energy being refracted and diffused radially outwardly. Such a tip will not exhibit the desired broad and uniform radiation patterns. At the other extreme, increasing the surface roughness beyond the preferred limits results in the tissue becoming entangled in the concaved recesses when the probe is inserted into the tissue thereby inhibiting withdrawal of the probe.

Operation of the present probe can best be understood with reference to Figure 4 where the laser beam coupled into the probe from the optic guide is shown to be internally impinging the roughened surface 12b of the probe tip. This laser energy is illustrated by rays 31 and 33. It will be noted that these rays do not arrive in parallel relationship, but rather, impinge from differing angles representative of the fact that each has travelled its own independent path including, in the general case, having undergone unpredictable internal reflections from the irregular surface thereabove.

Depending upon both the incident angle and the specific point of intersection of the incoming ray with the roughened surface 12b, the ray is either reflected internally, to again strike the surface 12b, or refracted externally to radiate from the probe. In the general case, the laser energy may, in fact, split; that is, some energy is reflected while the rest is refracted and radiated. Figure 4 depicts several representative ray tracks illustrating complete reflection, complete refraction, and a combination of both modes.

It is significant that the laser energy radiated from the probe is not directed in any particular direction, but rather, radiates in virtually all directions. In this manner, laser energy is emitted radially from the probe and, unlike conventional laser probes, can penetrate tissue lying radially adjacent the probe tip. It is, in short, this pseudo-random scattering or diffusing of laser energy which accounts for the significant radiation along the probe tip as depicted at 21 in Figure 6.

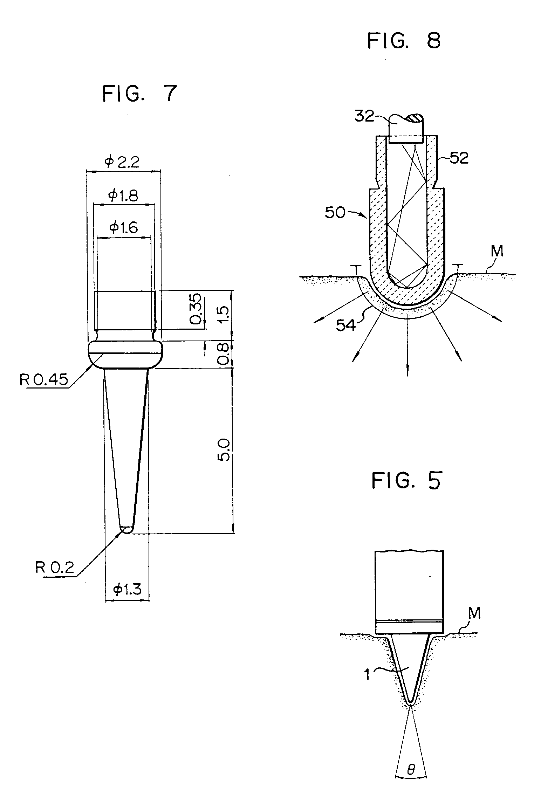

Figure 7 shows typical dimensions of one embodiment of the present probe. It will be appreciated that these dimensions are merely illustrative and the probe may advantageously be dimensioned according to specific treatment requirements. For example, the length of the laser diffusing portion 12 of the probe 10 may be suitably selected according to the insertion depth of the probe into the tissue M and it may in general be within a range of about 1.0 to 7.0 mm. Although the tip end of the laser diffusing portion 12 is not always required to be in semispherical shape, a pointed end would possibly be broken and therefore the tip end of the laser diffusing portion is preferably rounded.

In the embodiment described above, substantially the entire conically tapered portion functions to diffuse laser energy therefrom. Alternatively, any limited or fractional portion of this conical section, including the tip end 12a, may serve as a laser diffusing portion by correspondingly restricting the degree of surface roughness. The flange 16 as described before functions as an abutment or stop for positioning of the probe 10 in the tissue M when the probe 10 is injected into the tissue M until the forward end face of the projected flange 16 abuts against the tissue M. However, the flange 16 may of course be omitted as the case may be.

Figure 8 illustrates yet another embodiment of the present invention. Probe 50 has a substantially U-shaped cross-section and comprises a receiving portion 52 on the periphery of the base portion thereof for connection with the female connector 18 as described above. The outer and inner face of the probe have a semispherical shape and a frosted or roughened face is formed on the inner semispherical face and a part of a cylindrical portion adjacent to said inner semispherical face to provide a laser diffusing portion 54. Another portion of the inner face and the outer face remain smooth. The optical fiber 32 is disposd in the receiving portion of the probe 50. Most of the laser beam radiated from the tip end face of the optical fiber 32 enters the laser diffusing portion 54 where it is diffused and thereafter radiated from the outer surface of the tip end portion. This probe, which is brought into contact with the tissue rather than being inserted therein, is utilized for local hyperthermia, coagulation or photochemical therapy.

## Claims

1. A medical laser probe (10) for conveying laser energy from the output end of an optical laser waveguide (32) to a tissue undergoing laser treatment, the probe including a tip portion having a laser energy input region (38) for receiving laser energy from the optical waveguide and a laser energy radiation surface (12 b), said probe tip comprising a laser transmissive material (12), the laser energy radiation surface terminating in a semispherical portion (12a) at the extreme end of the probe tip, the laser transmissive material being devoid of light diffusing inclusions, characterised in that a selected one of the laser energy input region and the radiation surface define an irregular and uneven contour defined by recesses having a depth of between 10 and 60 microns, said recesses being formed by a grinding process for diffusing laser energy incident thereon whereby the laser radiation from the probe defines a broad highly diffused pattern.

2. A medical probe as claimed in claim 1 characterised by a flange (16) thereon, the radiation surface (12 a,b) extending from the flange, whereby said flange serves as a stop limiting the degree of insertion of the probe into tissue undergoing treatment.

3. A medical probe as claimed in claim 1 or 2, characterised in that the radiation surface (12 a,b) defines a conical shape, whereby the probe may be inserted into tissue undergoing treatment by first advancing the narrowed region of the conical surface into the tissue.

4. A medical laser probe as claimed in claim 1 characterised in that the laser transmissive material (52) surrounds the output end of said optical waveguide (32) thereby forming said laser energy receiving probe surface and said laser energy radiation probe surface, the receiving probe surface defining said irregular and uneven contour.

5. A medical laser probe as claimed in claim 1, characterised in that said laser transmissive material (52) surrounds the tip end of said optical waveguide (32) thereby forming a hollow inside region between the end of the optical laser waveguide and an inside surface of the probe, the inside surface having defining said irregular and uneven contour.

6. A medical probe as claimed in claims 5, wherein the outer surface of said probe is smooth.

## Patentansprüche

1. Medizinische Lasersonde (10) zum Übertragen von Laserenergie vom Ausgabeende eines optischen Laserwellenleiters (32) zu einem Laserbehandlung untervorfenen Gewebe, wobei die Sonde einen Spitzenabschnitt mit einem Laserenergieeingabebereich (38) zum Empfang von Laserenergie aus dem optischen Wellenleiter und einer Laserenergieabstrahlungsfläche (12b) aufweist, wobei die Sondenspitze ein laserdurchlässiges Material (12) aufweist, die Laserenergieabstrahlungsfläche in einem halbsphärischen Abschnitt (12a) an einem extremen Ende der Sondenspitze endet, das laserdurchlässige Material frei von lichtstreuenden Einschlüssen ist, dadurch gekennzeichnet, daß ein ausgewählter Laserenergieeingabebereich und die Abstrahlungsfläche eine unregelmäßige und ungleichmäßige Kontur bilden, definiert durch Vertiefungen mit einer Tiefe zwischen 10 und 60 Mikron - wobei die Vertiefungen durch einen Schleifprozess gebildet wurden, um diese auftreffende Laserenergie zu verteilen - wodurch die Laserstrahlung von der Sonde ein breites, hochdiffuses Muster bildet.

2. Medizinische Sonde, wie in Anspruch 1 beansprucht, gekennzeichnet durch einen Elansch (16) auf derselben, wobei die Abstrahlungsoberfläche (12a,b) sich vom Flansch aus erstreckt, wodurch der Flansch als Anschlag, der den Grad der Einführung der Sonde in behandelndes Gewebe bildet, dient.

3. Medizinische Sonde wie in Anspruch 1 oder 2 beansprucht, dadurch gekennzeichnet, daß die Abstrahlungsoberfläche (12a, b) eine konische Form bildet, wodurch die Sonde in zu behandelndes Gewebe eingeführt werden kann, indem zunächst der sich verjüngende Abschnitt der Konischen Oberfläche in das Gewebe eingeführt wird.

4. Medizinische Lasersonde, wie in Anspruch 1 beansprucht, dadurch gekennzeichnet, daß das laserdurchlässige Material (52) das Ausgabeende des optischen Wellenleiters (32) umgibt, wodurch eine Laserenergie aufnehmende Sondenoberfläche und die Laserenergie-Abstrahlungssondenoberfläche gebildet werden, wobei die aufnehmende Sondenoberfläche die unregelmäßige und ungleichmäßige Kontur definiert.

5. Medizinische Lasersonde, wie in Anspruch 1 beansprucht, dadurch gekennzeichnet, daß das laserdurchlässige Material (52) das spitze Ende des optischen Wellenleiters (32) umgibt, wodurch ein hohler Innenbereich zwischen dem Ende des optischen Laserwellenleiters und einer Innenoberfläche der Sonde gebildet wird, wobei die Innenoberfläche die unregelmäßige und ungleichmäßige Kontur begrenzt.

6. Medizinische Sonde, wie in Anspruch 5 beansprucht, wobei die äußere Oberfläche der Sonde glatt ist.

## Revendications

1. Sonde laser médicale (10) destinée à transmettre une énergie laser depuis l'extrémité de sortie d'un guide d'ondes laser optique (32) vers un tissu subissant un traitement laser, la sonde incluant une portion en pointe présentant une zone d'entrée de l'énergie laser (38) destinée à recevoir l'énergie laser depuis le guide d'ondes optique et une surface de rayonnement de l'énergie laser (12b), ladite pointe de la sonde comprenant une matière (12) de transmission du laser, la surface de rayonnement de l'énergie laser se terminant en une portion semi-sphérique (12a) à l'extrémité terminale de la pointe de la sonde, la matière de transmission du laser étant dépourvue d'inclusions diffusant la lumière, caractérisée en ce que l'une des zones d'entrée de l'énergie laser sélectionnée et la surface de rayonnement définissent un contour irrégulier et inégal défini par des évidements présentant une profondeur comprise entre 10 et 60 microns, lesdits évidements étant formés par un procédé de ponçage, de manière à diffuser une énergie laser agissant sur ceux-ci, le rayonnement laser provenant de la sonde définissant de ce fait une large image diffuse.

2. Sonde médicale selon la revendication 1, caractérisée par une bride (16) sur celle-ci, la surface de rayonnement (12a, b) s'étendant depuis la bride, ladite bride servant de ce fait de dispositif d'arrêt limitant le degré d'insertion de la sonde dans le tissu subissant un traitement.

3. Sonde médicale selon la revendication 1 ou 2, caractérisée en ce que la surface de rayonnement (12a, b) définit une forme conique, la sonde pouvant de ce fait être insérée dans le tissu subissant un traitement en avançant d'abord la zone rétrécie de la surface conique dans le tissu.

4. Sonde laser médicale selon la revendication 1, caractérisée en ce que la matière de transmission du laser (52) entoure l'extrémité de sortie dudit guide d'ondes optique (32), formant de ce fait ladite surface de la sonde recevant l'énergie laser et ladite surface de la sonde de rayonnement de l'énergie laser, la surface de la sonde recevant l'énergie définissant ledit contour irrégulier et inégal.

5. Sonde laser médicale selon la revendication 1, caractérisée en ce que ladite matière de transmission du laser (52) entoure l'extrémité en pointe dudit guide d'ondes optique (32), formant de ce fait une zone interne creuse entre l'extrémité du guide d'ondes laser optique et une surface interne de la sonde, la surface interne définissant ledit contour irrégulier et inégal.

6. Sonde médicale selon la revendication 5, dans laquelle la surface externe de ladite sonde est lisse.
